Europäisches Patentamt

European Patent Office

Office européen des brevets

(11) Numéro de publication: **0 410 904 A1**

## (12) DEMANDE DE BREVET EUROPEEN

(21) Numéro de dépôt: **90420343.7**

(22) Date de dépôt: **18.07.90**

(51) Int. Cl.5: **A61F 5/03**

(30) Priorité: **28.07.89 FR 8910508**

(43) Date de publication de la demande:
**30.01.91 Bulletin 91/05**

(84) Etats contractants désignés:
**AT BE CH DE DK ES GB GR IT LI LU NL SE**

(71) Demandeur: **GIBAUD, SOCIETE ANONYME**
**73, rue de la Tour**
**F-42000 Saint Etienne(FR)**

(72) Inventeur: **Pichon, Jean Claude**
**8, allée Jean Guitton**
**F-42000 Saint Etienne(FR)**

(74) Mandataire: **Perrier, Jean-Pierre et al**
**Cabinet GERMAIN & MAUREAU 12 rue de la**
**Républiqueque**
**F-42000 St-Etienne(FR)**

(54) **Bande ceinture lombo-abdominale.**

(57) Cette bande ceinture est du type composé d'une bande ceinture 2 réalisée en tissu, élastique au moins dans le sens longitudinal de la bande, et comportant des moyens 4-5 d'accrochage de ses extrémités de type velours à boucles et velours à crochets et dans sa partie ventrale, deux poches verticales 6, espacées par une distance correspondant à celle séparant les grands muscles droits ventraux et aptes chacune à recevoir une baleine.

Selon l'invention, elle comporte sur la face interne de sa partie dorsale, et le long de ses bords supérieur et inférieur, deux bandes 10 de velours à boucles aptes à coupèrer avec des bandes plus courtes 13 de velours à crochets constituant moyens de fixation amovible et de réglage en position dorsale pour un dossard 11, comportant au moins deux baleines verticales 16 espacées.

FIG.5

## BANDE CEINTURE LOMBO-ABDOMINALE.

L'invention est relative à une bande ceinture lombo-abdominale.

Le soutien abdominal après une opération ou en cas d'insuffisance musculaire est en général assuré par des ceintures ou corsets réalisés sur mesure, donc onéreux, et dont le port est peu agréable.

La présente invention a pour but de fournir une ceinture qui, pouvant assurer, simultanément mais aussi séparément un soutien abdominal, temporaire ou définitif, et un soutien lombaire, soit peu onéreuse, de pose commode, d'entretien aisé et de port non contraignant.

Cette ceinture est du type composée d'une bande en tissu, élastique au moins dans le sens longitudinal de cette bande, et comportant des moyens d'accrochage de ses extrémités et dans sa partie ventrale, deux poches verticales espacées par une distance correspondant à celle séparant les grands muscles droits ventraux et aptes chacune à recevoir une baleine.

Selon l'invention, cette bande ceinture comporte, sur la face interne de sa partie dorsale et le long de ses bords supérieurs et inférieurs, deux bandes de velours à boucles, aptes à coopérer avec des bandes plus courtes de velours à crochets constituant moyens de fixation amovibles et de réglage de la position dorsale d'un dossard baleiné comportant deux baleines verticales espacées.

Par les baleines de sa partie antérieure, cette bande ceinture assure un excellent soutien abdominal, complété éventuellement par un soutien lombaire assuré par le dossard baleiné.

En raison de son mode de fixation, ce dossard baleiné est amovible, ce qui permet d'utiliser la ceinture sans lui, mais aussi de le positionner parfaitement par rapport à la colonne vertébrale en ajustant sa position à la demande, en fonction de la tension communiquée à la bande ceinture et de la dimension morphologique du porteur.

Dans une forme d'exécution, cette bande ceinture comprend également une patte hypogastrique, constituée par une bande de tissu élastique, plus ferme que la bande ceinture, fixée en son milieu par une couture verticale à la base avant de la partie ventrale et entre les baleines verticales et en formant un V ouvert vers le haut, les extrémités libres de cette bande étant munies de moyens d'accrochage, de type velours à crochets, aptes à coopérer avec des plages de velours à boucles fixées sur la bande ceinture.

Ce mode de fixation des extrémités des fragments de bandes hypogastriques permet, non seulement, de régler la tension communiquée aux bandes, mais aussi leur inclinaison, de manière à obtenir un soutien ventral parfait.

D'autres caractéristiques et avantages ressortiront de la description qui suit en référence au dessin schématique annexé représentant à titre d'exemples non limitatifs une forme d'exécution de cette bande ceinture.

Figures 1 et 2 en sont des vues en plan montrant la bande ceinture à l'état développé et respectivement sa face externe et sa face interne,

Figure 3 est une vue de côté en élévation avec coupe partielle du dossard baleiné,

Figures 4 et 5 sont des vues en perspective montrant la bande ceinture en position d'utilisation, respectivement sans dossard baleiné et avec dossard baleiné.

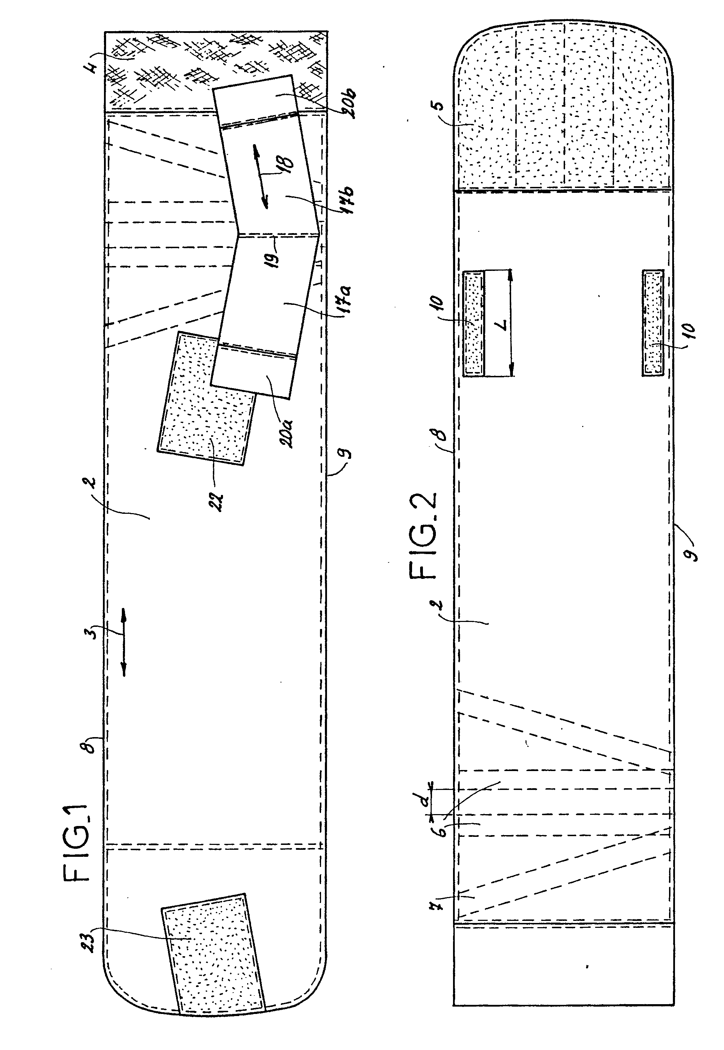

Comme montré aux figures 1 et 2, cette bande ceinture est constituée par une bande 2 en tissu, élastique au moins longitudinalement dans le sens de la flèche 3 et comportant, à l'une de ses extrémités, une nappe large 4 de velours à crochets dont les crochets sont externes et, à son autre extrémité, une nappe encore plus large 5 de velours à boucles, dont les boucles sont tournées du côté de sa face interne. Sur sa partie ventrale, c'est à dire sur sa partie qui normalement doit venir sur le ventre de l'utilisateur, la ceinture 2 comprend deux poches verticales 6 espacées par une distance d, correspondant sensiblement à la distance séparant les grands muscles droits ventraux, et deux poches latérales et obliques 7 formant un V ouvert vers le haut. Chacune de ces poches est destinée à recevoir une baleine, amovible ou non.

Comme le montre la figure 2, sur la face interne de sa partie dorsale et le long de ses bords supérieurs 8 et 9, la bande ceinture 2 comporte deux nappes 10 de velours à boucles de longueur L. Ces nappes sont destinées à coopérer avec des bandes 13 de velours à crochets de longueur l fixées à proximité des bords, respectivement supérieur et inférieur, d'un dossard 14 muni de deux poches 15 espacées pour des baleines 16. La différence de longueur entre les bandes 13 et 10 permet d'ajuster le positionnement du dossard sur la ceinture, comme cela sera expliqué plus loin.

Enfin, dans la forme d'exécution représentée, la bande ceinture est solidaire d'une bande hypogastrique 17a-17b réalisée dans un tissu, élastique dans le sens de la flèche 18 mais plus ferme que le tissu constituant la bande ceinture 2. Cette bande est fixée à mi longueur et par une couture 19 sur la face avant de la partie ventrale, sensiblement au milieu de l'intervalle entre les poches verticales 6 et à proximité du bord inférieur 9 de la ceinture. Chaque fragment de bande 17a ou 17b est muni à son extrémité libre d'une bande 20a ou 20b de velours à crochets dont les crochets sont tournés en direction de la ceinture. La bande 20a est destinée à coopérer avec une nappe 22 de velours à boucles fixée sur la face externe de la ceinture, tandis que la bande 20b est; destinée à coopérer avec une nappe de velours à boucles 23 fixée sur la même face externe de la ceinture, mais à l'extrémité libre de celle-ci.

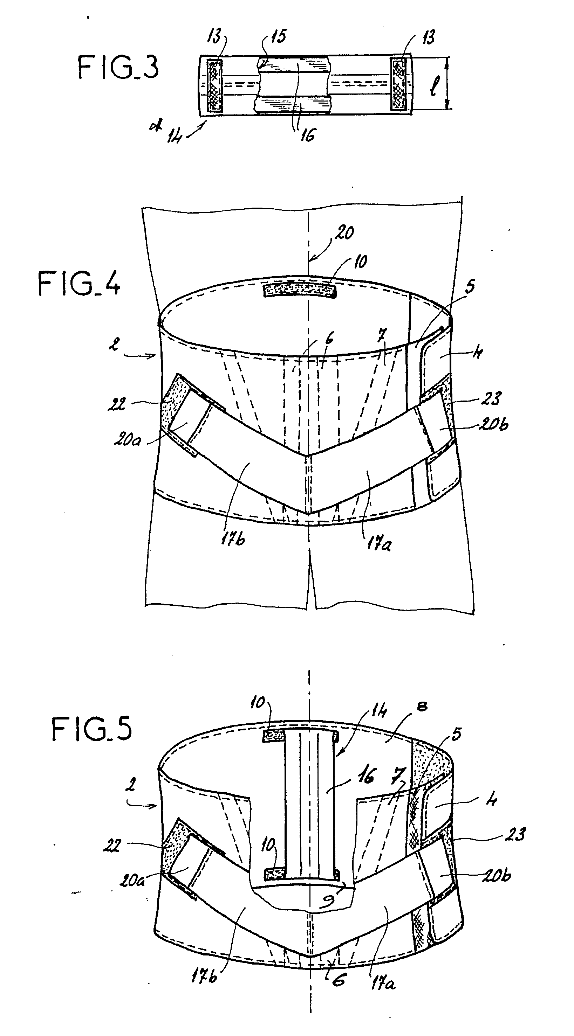

De façon connue, lorsque la ceinture est placée par l'utilisateur, sa fixation est assurée par accrochage des crochets de la bande extrême 4 sur la nappe 5, comme représenté aux figures 4 et 5. Ces figures montrent également que pour assurer un bon maintien ventral, la ceinture doit être positionnée de manière que les deux baleines verticales 6 soient de part et d'autre du milieu du ventre. Le réglage des pattes hypogastriques 17a et 17b, en inclinaison et en tension, est facilité par la grande dimension des nappes 22 et 23.

Lorsque la ceinture est utilisée avec le dossard 14, avant de procéder au réglage de la tension des pattes hypogastriques, mais après réglage personnalisé de la tension de la ceinture, l'opérateur positionne le dossard. Cette opération s'effectue très simplement, après réglage normal de la ceinture sur le sujet, en repérant à l'aide de deux doigts, pinçant la nappe 10, la position de la colonne vertébrale, schématisée par l'axe 20 figure 4 puis, en maintenant pincés les doigts et en ouvrant; la ceinture, en appliquant sur les deux nappes 10 les bandes 13, en prenant soin de faire coïncider l'intervalle entre les deux baleines 16 avec la zone pincée par les doigts. Une fois cette opération effectuée et après remise en place de la ceinture autour de l'abdomen, le seul positionnement des baleines verticales 6 par rapport au plan médian du ventre, assure automatiquement le positionnement des baleines 16 du dossard 11 de part et d'autre de la colonne vertébrale.

Ainsi, une telle ceinture permet, après réglage personnalisé de la tension de cette ceinture, d'effectuer le réglage du positionnement du dossard afin que la position de celui-ci soit parfaitement adaptée à la morphologie du porteur en tenant compte de la tension de la ceinture. En d'autres termes, lors d'une nouvelle mise en place de la ceinture, si le porteur prend soin de bien placer les baleines ventrales et le dossard, il est certain, en fermant la ceinture d'obtenir sur celle-ci la tension optimale.

Cette bande ceinture est beaucoup moins onéreuse que les corsets réalisés sur mesure par des orthopédistes et peu contraignante au port, tout en assurant un excellent soutien abdominal, associé à un soutien lombaire.

Une telle ceinture peut être utilisée pour procurer un soutien abdominal temporaire après intervention chirurgicale, après grossesse, mais aussi pour assurer un soutien abdominal définitif après éventration, en présence d'une hernie ombilicale, en cas d'obésité ou pour renforcer la paroi en cas de paralysie abdominale.

La mise en place du dossard baleiné procure un maintien abdomino-vertébral lorsqu'une pathologie lombaire est associée à une faiblesse de la paroi abdominale ou à une pathologie abdominale telle que paralysie, hernie, éventration.

## Revendications

1. Bande ceinture lombo-abdominale du type composé d'une bande ceinture (2) réalisée en tissu, élastique au moins dans le sens longitudinal de la bande, et comportant des moyens d'accrochage de ses extrémités, de type velours à boucles et velours à crochets, et,dans sa partie ventrale, deux poches verticales (6), espacées par une distance correspondant à celle séparant les grands muscles droits ventraux et aptes chacune à recevoir une baleine, caractérisée en ce qu'elle comporte, sur la face interne de sa partie dorsale et le long de ses bords supérieur (8) et inférieur (9), deux bandes (10) de velours à boucles aptes à coopérer avec des bandes plus courtes (13) de velours à crochets constituant moyens de fixation amovible et de réglage de la position dorsale d'un dossard baleiné (11), comportant au moins deux baleines verticales (16) espacées.

2. Bande ceinture selon la revendication 1, caractérisée en ce qu'elle comprend une patte hypogastrique constituée par une bande de tissu élastique (17a-17b), plus ferme que la bande ceinture, fixée en son milieu à la base de la face avant de la partie ventrale par une couture verticale (19), bande dont les deux fragments forment un V ouvert vers le haut, les extrémités libres de chacun de ces fragments (17a-17b) étant munies de moyens d'accrochage (20a-20b), de type velours à crochets, aptes à coopérer avec des plages (22-23), de velours à boucles, fixées sur la face externe de la bande ceinture.

EP 0 410 904 A1

# FIG.1

# FIG.2

FIG.3

FIG.4

FIG.5

5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-1 612 121  (HITTENBERGER) <br> * En entier * <br> --- | 1,2 | A 61 F    5/03 |
| A | FR-A-2 114 033  (ESSWEIN) <br> * Revendications; figures 2,4 * <br> --- | 1 | |
| A | FR-A-  486 768  (BAUGATZ) <br> * Page 2, lignes 16-35; figure 1 * <br> --- | 1 | |
| A | FR-A-2 106 247  (TEMOVA ETABLISSEMENT) <br> * Page 3, lignes 5-12; figures 3,4 * <br> --- | 2 | |
| A | FR-A-2 589 723  (LEVEILLE) <br> ----- | | |

**DOMAINES TECHNIQUES RECHERCHES (Int. Cl.5)**

A 61 F

Le présent rapport a été établi pour toutes les revendications

| Lieu de la recherche | Date d'achèvement de la recherche | Examinateur |
|---|---|---|
| LA HAYE | 17-10-1990 | SANCHEZ Y SANCHEZ J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)